# EUROPÄISCHE PATENTSCHRIFT

(11) **EP 3 513 777 B1**
(45) Veröffentlichungstag und Bekanntmachung des Hinweises auf die Patenterteilung: **04.10.2023**
(21) Anmeldenummer: 18152850.6
(22) Anmeldetag: 22.01.2018
(51) Int. Cl.: A61K 6/17, A61K 6/20

(54) **VERFAHREN ZUR REMINERALISATION VON ZÄHNEN**
METHOD FOR THE REMINERALISATION OF TEETH
PROCÉDÉ DE REMINÉRALISATION DES DENTS

(43) Veröffentlichungstag der Anmeldung: 24.07.2019
(73) Patentinhaber: Ivoclar Vivadent AG, 9494 Schaan (LI)
(72) Erfinder: Lendenmann, Urs, 9472 Grabs (CH); Bolis, Carlo, 7206 Igis (CH)
(74) Vertreter: Uexküll & Stolberg

(56) Entgegenhaltungen:
- EP-A1- 0 831 764
- EP-B1- 0 831 764
- WO-A1-2008/068149
- WO-A1-2012/145619
- WO-A1-2014/151244
- DE-A1-102016 204 106
- US-A- 4 556 561
- BEATRIZ M. SOUZA ET AL: "Effect of an Experimental Paste with Hydroxyapatite Nanoparticles and Fluoride on Dental Demineralisation and Remineralisation in situ", CARIES RESEARCH, Bd. 49, Nr. 5, 2. November 2015 (2015-11-02), Seiten 499-507, XP055487064, CH ISSN: 0008-6568, DOI: 10.1159/000438466

## Beschreibung

Die vorliegende Erfindung betrifft Zusammensetzungen zur Remineralisation von Zähnen. Dabei wird zuerst eine Lösung einer Fluoridkomponente auf die Zähne aufgetragen und anschließend ein Sol oder ein Kolloid einer nano-Calciumkomponente aufgetragen. Dies führt anschließend zu einer schnellen Remineralisation von Zahnhartsubstanz.

### Stand der Technik

Karies von Zähnen beginnt, wenn Säure dem Hydroxylapatitanteil der Zahnhartsubstanz Mineralien entziehen. Dies reduziert die Dichte des Minerals und erhöht die Durchlässigkeit der Zahnstruktur für Flüssigkeiten und Ionen. Die Säure kann direkt durch Nahrung, vor allem saure Getränke auf die Zähne gelangen. Von größerer Bedeutung sind aber Kohlenhydrate, vor allem Saccharose, welche von Biofilmen auf den Zähnen zu organischen Säuren, vor allem Milchsäure fermentiert werden. Dabei kann der pH im Biofilm auf den Zähnen in wenigen Minuten auf ca. 4 - 5 absinken (Lingstrom et al., 1993, J Dent Res, 72:865-870). Wenn eine Untersättigung in Bezug auf die Löslichkeit von Hydroxylapatit im Speichel vorherrscht, dann bilden sich als Kariesvorstufe (Initialkaries) zuerst Entkalkungen. Diese sind makroskopisch als weisse Flecken (white spot) erkennbar (Arends & Christoffersen, 1986, J Dent Res, 65:2-11). Solange die initialen Kariesläsion nicht so weit fortgeschritten ist, dass ein Loch entsteht, kann sie remineralisiert werden.

Obwohl die Möglichkeit der Remineralisation von Schmelzprüfkörpern im Labor schon vor 1970 nachgewiesen wurde (Johansson, 1965, J Dent Res, 44:64-70; Feagin et al., 1969, Arch Oral Biol, 14:1407-1417), gibt es bis heute keine Produkte, die klinisch nachweisbar und voraussagbar besser remineralisieren als Fluoride. Tatsächlich wurde aber schon vor vielen Jahren beschrieben, dass *in vivo* Remineralisation wesentlich langsamer verläuft als im Labor (Gelhard & Arends, 1984, J Biol Buccale, 12:49-57).

In der Prävention von Karies haben sich bis jetzt vor allem gute Mundhygiene und die topische Applikation von Fluorid auf die Zähne bewährt. Fluorid reduziert die Löslichkeit von Hydroxylapatit. Regelmässige Fluoridapplikation auf Zähen führt auch zu Remineralisation (Gao et al. 2016, BMC Oral Health, 16:12). Dies benötigt jedoch sehr viel Zeit und der Erfolg ist schlecht vorhersagbar. Verfahren zur Remineralisation, welche in kurzer Zeit zu substanzieller Remineralisation führen, bedeuten darum eine neue Behandlungsmöglichkeit in der Zahnmedizin.

Die WO 2012/145619 A1 beschreibt die Synthese von Calciumfluorid-, Dicalciumphosphat-, Hydroxylapatit- und Fluorapatit-Nanopartikeln. Diese sollen sich zur Behandlung der Hypersensibilität von Zähnen eignen. Hierzu werden die Zähne mit einer Suspension oder einem Gel behandelt, das einen oder mehrere dieser nanopartikulären Stoffe enthält. Die Nanopartikel sollen dabei in die Dentintubuli eindringen und diese dabei zumindest teilweise verschließen.

Die DE 10 2015 102 156 A1 beschreibt eine Methode zur Bereitstellung einer Zink- und/oder Erdalkalimetall-Seltenerdmetall Fluorid Sol Lösung. Das Sol soll sich zur Prävention bzw. Behandlung kariöser Läsionen eignen und sich in Füllungsmaterialien integrieren lassen.

Die US 4,397,837 beschreibt ein Verfahren zur Remineralisation von Zähnen, bei dem gleichzeitig oder nacheinander eine Lösung eines wasserlöslichen Calciumsalzes und eine Lösung eines wasserlöslichen Phosphats, die Fluorid enthalten kann, in Kontakt mit den Zähnen gebracht werden. Die Stoffe sollen in den demineralisierten Zahn eindringen und dort ein Präzipitat bilden.

Die US 4,556,561 beschreibt Zusammensetzungen und Verfahren für die topische Fluoridierung und Remineralisation von Zahngewebe. Hierzu wird eine nichtwässrige Dispersion eingesetzt, die Hydroxylapatit und Calciumdihyrogenphosphatdihydrat oder Calciumdiyhdrogenphosphat enthält und die mit Fluorapatit oder Calciumfluorid gesättigt ist. Vorzugsweise wird eine weitere Komponente aufgetragen, die ein Fluorid enthält, wie angesäuertes Phosphatfluorid, Zinnfluorid, Natriumfluorid oder Titantetrafluorid.

Die US 5,895,641 offenbart ein Verfahren zur Remineralisation von Zähnen. Dabei wird zuerst eine Komponente dosiert, welche 0,05 - 15 Gew.-% Calciumchlorid oder Calciumnitrat enthält. Danach wird eine zweite Komponente dosiert, welche 0,5-15 Gew.-% eins löslichen Phosphatsalzes und 0,01 - 5,0 Gew.-% eines löslichen Fluoridsalzes beinhaltet. Danach werden die Komponenten mit Wasser oder Speichel gemischt, so dass sich ein pH zwischen 4,5 und 10 einstellt. Unmittelbar danach werden die Komponenten auf den Zahn aufgebracht.

Die US 5,858,333 beschreibt ein 2-Komponenten Mundpflegeprodukt, welches Initialläsionen in Zähnen oder exponierte Dentintubuli mineralisieren soll. Die erste kationische Komponente enthält ein wasserlösliches Calciumsalz in einem pharmazeutisch akzeptablen Trägermaterial. Die zweite anionische Komponente enthält ein wasserlösliches Phosphatsalz in einem pharmazeutisch akzeptablen Trägermaterial. Das Trägermaterial einer der Komponenten ist wässrig und das der anderen nichtwässrig aber hydrophil. Die beiden Komponenten werden vor der Anwendung miteinander gemischt.

Die WO 98/43602 offenbart zweikomponentige Mittel zur Remineralisation von Zähnen, die eine kationische Komponente A und eine anionische Komponente B enthalten. Die kationische Komponente A enthält eine ganz oder teilweise wasserlösliche Calciumverbindung und die anionische Komponente B eine wasserlösliche Fluoridverbindung. Die Komponenten werden vor dem Aufbringen auf die Zähne miteinander gemischt. Die calciumhaltige Komponente enthält zusätzlich eine wasserlösliche Magnesiumverbindung, die nach dem Mischen der Komponenten die Reaktion zwischen der Calcium- und der Fluorverbindung verzögern soll.

Die DE 102 23 157 C1 offenbart ein Verfahren zur Ausbesserung von Defekten an Zahnmaterial. Hierzu werden ein Gelatinegel, das Phosphationen und optional Fluoridionen enthält, und ein sog. Schutzgel, das frei von Phosphationen ist, sequentiell auf den Zahn aufgetragen. Anschließend wird der Zahn mit einem Calciumionen enthaltenden Medium behandelt. Hierzu wird der Zahn in den Ausführungsbeispielen für mehrere Tage in einer Calciumchloridlösung gelagert. Das Verfahren soll das Aufwachsen einer Apatitschicht auf der Zahnoberfläche bewirken.

Die EP 1 645 263 A1 beschreibt ein Mundpflegeprodukt, das eine fluoridhaltige Zusammensetzung A und eine calciumhaltige Zusammensetzung B umfasst. Die Komponente A enthält zusätzlich eine anorganische Phosphorsäure oder ein Salz davon. Die Komponente B enthält das Calciumsalz einer organischen Säure mit einem pK-Wert von 3 bis 11. Die beiden Komponenten werden nacheinander auf den Zahn aufgetragen, wo sie unter Bildung von Calciumfluorid oder Calciumphosphat miteinander reagieren sollen. Hierdurch soll die Aufnahme von Calciumfluorid und Calciumphosphat in den Zahn verbessert werden.

Die WO 2008/068149 A1 beschreibt ein Mundpflegeprodukt, das eine erste Zusammensetzung, die ein unlösliches Calciumsalz enthält, und eine zweite Zusammensetzung umfasst, die eine Quelle von Phosphationen enthält. Die erste Zusammensetzung enthält vorzugsweise weder Phosphat- noch Fluoridionen. Das unlösliche Calciumsalz soll auf der Zahnoberfläche *in situ* durch die Phosphationen zu Hydroxylapatit umgewandelt werden.

Die US 2,802,268 beschreibt ein Verfahren zum Verschließen von Randspalten zwischen Zahn und Füllung bei dem zuerst ein lösliches Silikofluorid wie MgSiF₆ in den Spalt gebracht und anschließend durch eine alkalische Flüssigkeit wie z.B. eine Calciumhydroxidsuspension die Präzipitation von Siliciumdioxid, Magnesiumfluorid und Calciumfluorid bewirkt wird.

Die EP 2 676 938 A1 offenbart ein Verfahren zur Herstellung eines Calciumfluoridsols bei dem eine Calciumverbindung in einem nicht-wässrigen Lösungsmittel mit einer nicht-wässrigen Fluorwasserstofflösung versetzte wird. Als Calciumverbindung werden vorzugsweise die Salze organischer Säuren eingesetzt, wie z.B. Calciumacetat oder Calciumlactat. Bevorzugte nichtwässrige Lösungsmittel sind Alkohole wie z.B. Methanol oder Ethanol. Die Sole sollen sich zur Herstellung von Antireflexbeschichtungen auf Glas eignen.

Die WO 02/20696 A1 beschreibt die Synthese von Metallsalz-Nanopartikeln. Die Nanopartikel können fluoreszierende Eigenschaften haben. Bei diesem Verfahren wird ein Metallsalz als Kationenquelle zusammen mit einer Anionenquelle und einer das Kristallwachstum steuernden Komponente in einem organischen Lösungsmittel dispergiert oder gelöst und dann bei einer vorgegebenen Temperatur gehalten. Als Metallsalze eignen sich unter anderem Calciumsalze.

Die WO 2014/1512244 A1 offenbart eine Zusammensetzung und Kit zur Remineralisation von Zähnen enthaltend ein Primer zur Vorbehandlung des Zahns und eine nano-Calciumkomponente. Die Zusammensetzung kann auch eine fluoridhaltige Komponente enthalten.

### Kurzbeschreibung der Erfindung

Der Erfindung liegt die Aufgabe zu Grunde, Zusammensetzungen zur Verfügung zu stellen, die sich zur Remineralisation von Zähnen, zur Behandlung initialer Kariesläsionen, zum Kariesschutz und zur Verhinderung und Behandlung von Zahnerosionen eignen.

Die Aufgabe wird durch eine Zusammensetzung gelöst, wie in Anspruch 1 definiert, die eine fluoridhaltige Komponente und eine calciumhaltige Komponente umfasst, wobei man zunächst eine Lösung einer fluoridhaltigen Komponente und dann ein Sol einer calciumhaltigen Komponente in einem volatilen Lösungsmittel auf die zu behandelnde Zahnoberfläche aufbringt. Gemäß einer bevorzugten Ausführungsform wird die Zahnoberfläche vor dem Aufbringen der Fluoridkomponente einer optionalen Vorbehandlung unterworfen, beispielsweise einer Säurebehandlung und/oder einer Zahnreinigung.

### Detaillierte Beschreibung der Erfindung

Die Erfindung betrifft eine Zusammensetzung zur Remineralisation von Zähnen, insbesondere zur Behandlung initialer Kariesläsionen, beispielsweise von Fissuren, Glattflächen, Interdentalflächen und Zahnhälsen, zum Kariesschutz an ausgewählten Zahnflächen, und zur Verhinderung und Behandlung der Zahnerosionen.

Die Verwendung der Zusammensetzung umfasst die folgenden Schritte:
(i) Optionale Vorbehandlung des Zahns,
(ii) Applikation einer Fluoridkomponente und
(iii) Applikation einer nano-Calciumkomponente.

Die nanopartikuläre Calciumkomponente bildet nach dem Aufbringen auf der Zahnoberfläche eine feste Schicht aus.

Die fluoridhaltige Komponenten und die calciumhaltige Komponente liegen in räumlich getrennter Form vor.

### I. Optionale Vorbehandlung des Zahns

Vorzugsweise werden die zu behandelnden Zahnareale vor der eigentlichen Behandlung gereinigt. Dabei werden Zahnstein (Kalkulus) und andere Beläge auf den Zähnen entfernt. Besonders bevorzugt wird eine professionelle Zahnreinigung durchgeführt.

Da nach einer (professionellen) Zahnreinigung mit einer abschliessenden Politur ein dünner Film von Debris, die sogenannte Schmierschicht, auf dem Zahn verbleiben kann, ist es bevorzugt, die zu behandelnden Zahnareale etwas zu ätzen, damit die anschließende Fluoridkomponente tief in den Zahnschmelz eindringen kann. Dazu wird ein Ätzmittel punktuell auf die betreffenden Bereiche, insbesondere die demineralisierten, als weisse Flecken sichtbaren Initialläsionen, aufgebracht und für kurze Zeit darauf belassen. Danach wird es abgespült. Die Einwirkzeit der Säure ist nicht sehr kritisch. Sie sollte lang genug sein, um die Schmierschicht zu lösen, aber nicht so lang, dass der Zahnschmelz unnötig geschwächt wird. Erfindungsgemäß sind Zeiten zwischen 1 s und 1000 Sekunden, bevorzugt 5 s - 120 s, besonders bevorzugt 5 s - 60 s bevorzugt.

Als Säuren zum Ätzen kommen wässrige Lösungen von Phosphorsäure, Salzsäure, Salpetersäure, Schwefelsäure, Milchsäure, Essigsäure, Ameisensäure, Zitronensäure, Ethylendiamintetraessigsäure etc. in Frage. Bevorzugt sind Phosphorsäure und Milchsäure, besonders bevorzugt ist Phosphorsäure, vorzugsweise 37%-ige Phosphorsäure.

Damit diese Säurelösungen punktuell genau appliziert werden können, enthalten sie vorzugsweise einen Verdicker. Solche Ätzmittel sind an sich aus der dentalen Adhäsivtechnik bekannt und gut beschrieben. Bevorzugt ist 37%-iges Phosphorsäure-Ätzgel, wie z.B. das unter der Bezeichnung Total Etch von der Ivoclar Vivadent AG kommerziell erhältliche Gel.

Das Ätzmittel wird nach der gewünschten Einwirkzeit mit Wasser abgespült und der Zahn danach getrocknet, vorzugsweise mit einem Luftstrom, aber auch andere Methoden sind geeignet.

### II. Applikation Fluoridkomponente

Nach der Reinigung und/oder der Säurebehandlung wird die Fluoridkomponente auf den Zahn aufgebracht.

Es empfiehlt sich, im Sinne einer umfassenden Fluoridierung der Zähne nach einer Zahnreinigung, die Fluoridkomponente auf die ganze natürliche Bezahnung aufzutragen. Die Fluoridkomponente kann aber auch einfach nur auf die zu behandelnde Stellen aufgetragen werden, besonders dann, wenn vorgängig keine professionelle Zahnreinigung erfolgt ist. Wichtig für den Erfolg des Verfahrens ist jedoch, dass auf alle Flächen, auf die anschließend die Nano-Calciumkomponente aufgetragen wird, auch Fluoridlösung aufgebracht wurde.

Als Fluoridkomponente wird vorzugsweise eine Zusammensetzung verwendet, die eine oder mehrere der folgenden Fluoridverbindungen enthält: Natriumfluorid, Kaliumfluorid, Ammoniumfluorid, Ammoniumbifluorid, Natriummonofluorophosphat, Kaliummonofluorophosphat, Salze von Tetra- oder Hexafluoroanionen wie z.B. Ammoniumhexafluorosilikat, Magnesiumhexafluorosilikat, Kaliumhexafluorophosphat, Ammoniumhexafluorotitanat, Ammoniumhexafluoroaluminat. Zirkonfluorid, Tetra-n-butylammonium dihydrogentrifluorid (TBAF-3), Rubidiumfluorid, Cäsiumfluorid, Kaliumbifluorid (KHF₂), Silber-I-fluorid (AgF), Zinn-II-fluorid (SnF₂), Olaflur und Dectaflur. Besonders bevorzugt sind: Ammoniumfluorid, Ammoniumbifluorid, Kaliumfluorid, Tetra-n-butylammoniumdihydrogentrifluorid.

Die Fluoridverbindung(en) wird (werden) vorzugsweise in Form einer Lösung eingesetzt. Als Lösungsmittel sind Stoffe oder Stoffgemisch geeignet, welche die Fluoridverbindung zumindest teilweise und vorzugsweise vollständig zu lösen können.

Bevorzugte Lösungsmittel sind Wasser, Ethanol, Isopropanol, Aceton, Methanol und Propylenglycol oder Mischungen davon. Besonders bevorzugt sind Wasser, Ethanol, Isopropanol und Aceton oder deren Mischungen. Insbesondere für Tetra-n-butylammoniumdihydrogentrifluorid sind Aceton oder Mischungen von Aceton mit Wasser, Ethanol oder Isopropanol besonders bevorzugt.

Bevorzugt sind Lösungen, die mehr als 1500 ppm, bevorzugt mehr als 5000 ppm Fluorid (bezogen auf das Fluoridanion) in gelöster Form enthalten. Die maximale Konzentration hängt von der Wahl des Lösungsmittels und des Fluorsalzes ab. Sie liegt vorzugsweise unter 20 Gew.-%, besonders bevorzugt unter 10 Gew.-%.

Optional kann diese Fluoridlösung auch Säuren enthalten, damit Beläge entfernt werden, welche das Eindringen der anschließenden Komponenten behindern könnten. Als Säuren kommen insbesondere Phosphorsäure, Salzsäure, Salpetersäure, Schwefelsäure, Milchsäure, Essigsäure, Ameisensäure, Zitronensäure Ethylendiamintetraessigsäure etc. in Frage. Vorzugsweise enthält die Fluoridlösung kein Phosphat.

Nach dem Auftragen der Fluoridlösung sollten Überschüsse der Fluoridlösung möglichst entfernt werden. Dies kann durch Verblasen mit Luft, einem Sauger oder einem Tupfer erfolgen. Wenn keine Überschüsse der Fluoridlösung auf den Zähnen vorhanden sind, dann kann gleich weitergefahren werden. Die Zähne können nach dem Aufbringen der Fluoridlösung optional getrocknet werden.

### III. Applikation nano-Calciumkomponente

Nach der Fluoridkomponente wird die Nano-Calciumkomponente aufgetragen. Diese kann wiederum vollflächig auf das ganze Gebiss oder nur auf die zu behandelnden Areale aufgetragen werden. Nach dem Aufbringen bildet die Calciumkomponente eine Schicht, die den Zahn ganz oder teilweise bedeckt. Die Schichtbildung erfolgt beispielsweise beim Verdampfen des Lösungsmittels der Calciumkomponente. Hierbei verdichten sich die Nano-Calciumpartikel zu einer Schicht. Vorzugsweise wird die Schichtbildung durch das aktive Trocken der Calciumkomponente beschleunigt. Die Bildung einer Schicht kann aber auch auf andere Weise bewirkt werden, beispielweise durch die Zugabe eines Gelbildners zu der Calciumkomponente. Die Schichtbildung kann auch durch spontane Aggregation der Partikel erfolgen, beispielsweise dann, wenn das Sol mit Wasser oder Speichel in Kontakt kommt.

Die Calciumkomponente enthält ein Calciumsalz, vorzugsweise Calciumfluorid, Calciumcarbonat, Calciumsulfat Calciumsilikat, Calciumoxid oder Calciumhydroxid. Besonders bevorzugt sind Calciumfluorid, Calciumcarbonat, Calciumsulfat und Calciumsilikat, ganz besonders Calciumcarbonat und insbesondere Calciumfluorid. Erfindungsgemäß liegt das Calciumsalz in nanopartikulärer Form vor. Bevorzugt sind Nanopartikel mit einer Partikelgröße von < 100 nm, besonders bevorzugt < 40 nm und ganz besonders bevorzugt < 30 nm. Vorzugsweise liegt die Partikelgröße in einem Bereich von 1 bis 100 nm, besonders bevorzugt 1 bis 40 nm und ganz besonders bevorzugt 1 bis 30 nm.

Bei der Partikelgröße handelt es sich in allen Fällen, wenn nicht ausdrücklich anders angegeben, um den mittlerer Partikeldurchmesser (Zahlenmittel) gemessen mit dynamischer Lichtstreuung (DLS), vorzugsweise mit einem Malvern ZetaSizer.

Vorzugsweise enthält die Calciumkomponente kein Phosphat, insbesondere kein Calciumphosphat.

Das nanopartikuläre Calciumsalz liegt als Sol in einem volatilen Suspensionsmittel vor. Als Suspensionsmittel für die Calciumpartikel kommen insbesondere oral akzeptable Lösungsmittel in Frage, welche unter normalen Umgebungsbedingungen oder unter oralen Bedingungen in kurzer Zeit von selber verdampfen oder mit Hilfe eines Luftstroms eingetrocknet werden können. Bevorzugte Suspensionsmittel sind Alkohole, Ester, Ether, Ketone, Alkane, Alkene, Wasser oder Mischungen davon, insbesondere Ethanol, Methanol, n-Propanol, i-Propanol, n-Butanol, sek. Butanol, Isoamylalkohol, Aceton, Wasser, Acetonitril, Ethylacetat, Methoxypropanol, Dibutylether, Dioxan, Metylethylketon, Heptan, Hexan oder Dimethylformamid. Besonders bevorzugt sind Ethanol, Aceton, Isopropanol, Wasser und deren Mischungen. Organische Lösungsmittel oder Mischungen von organischen Lösungsmitteln und Wasser sind bevorzugt, besonders bevorzugt sind wasserfreie Lösungsmittel.

Ein wesentlicher Aspekt der Erfindung ist, dass die Calciumkomponente nanopartikuläre Partikel eines Calciumsalzes enthält. Calciumsalz sowie Art und Menge des Suspensionsmittels werden daher so gewählt, dass sich die Partikel nicht in dem Lösungsmittel auflösen. Sie müssen zumindest während der Anwendung in partikulärer Form vorliegen. Das nanopartikuläre Calciumsalz und das Suspensionsmittel können in getrennter Form vorliegen. In diesem Fall wird das Calciumsalz vor der Anwendung in dem Suspensionsmittel dispergiert. Bevorzugt sind gebrauchsfertige Suspensionen (Sole) des oder der Calciumpartikel.

Erfindungsgemäß sind Suspensionen bevorzugt, die einen pH-Wert von kleiner 11,0 und vorzugsweis von kleiner 10 aufweisen. Bei wasserfreien Suspensionen die Suspension zur Bestimmung des pH-Werts im Verhältnis von 1:1 mit Wasser gemischt. Vorzugsweise liegt der pH-Wert in einem Bereich von 4,4 bis 11, besonders bevorzugt 5 bis 10 und ganz besonders bevorzugt 6 bis 10.

Der Anteil des Calciumsaslzes im Sol oder Kolloid kann zwischen 0,0001 - 99,9999 Gew.-% liegen. Bevorzugt sind Anteile von 0,1 Gew.-% bis 40 Gew.-%, besonders bevorzugt 1 Gew.-% bis 30 Gew.-%, ganz besonders bevorzugt 5 Gew.-% bis 25 Gew.-% und insbesondere 6 Gew.-% bis 12 Gew.-%. Der Anteil sollte jedoch auch nicht zu hoch sein, damit das Sol oder Kolloid dünnflüssig genug bleibt, um es mit einem Pinsel oder einem Bürstchen auf den Zahn auftragen zu können.

Nach dem Aufbringen des Sols auf die Zahnoberfläche bilden die Nanopartikel eine feste Schicht auf der Zahnoberfläche. Die Schichtbildung erfolgt vorzugsweise durch Verdampfen des Lösungsmittels, wobei das Trocknen aktiv beschleunigt werden kann, beispielsweise durch Verblasen des Lösungsmittels mit einem Luftstrahl. Nach dem Trocken kann die getrocknete Schicht auf dem Zahn verbleiben. Sie wird im Laufe der Zeit, beispielsweise beim Zähneputzen, abgetragen. Es ist aber auch möglich, die Schicht nach der Behandlung aktiv zu entfernen.

Im Folgenden wird die Erfindung anhand von Figuren und Beispielen näher erläutert.

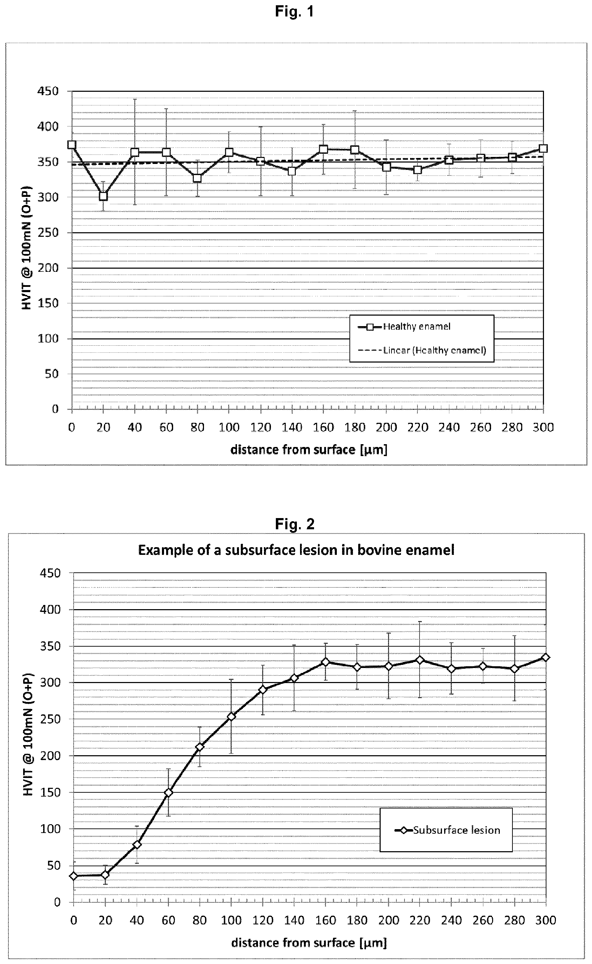

Fig. 1 zeigt das Härteprofil eines unbehandelten Zahns. Die Härte des Zahn an der Oberfläche (distance = 0 µm) entspricht der Härte in tieferen Bereichen (distance = 300 µm).
Fig. 2 zeigt das Härteprofil des Zahns nach Lagerung in einer Demineralisationslösung. Die Demineralisation des Zahns ist mit einer deutlichen Abnahme der Härte verbunden, die sich besonders an der Zahnoberfläche zeigt, die unmittelbar in Kontakt mit der Demineralisationslösung steht.

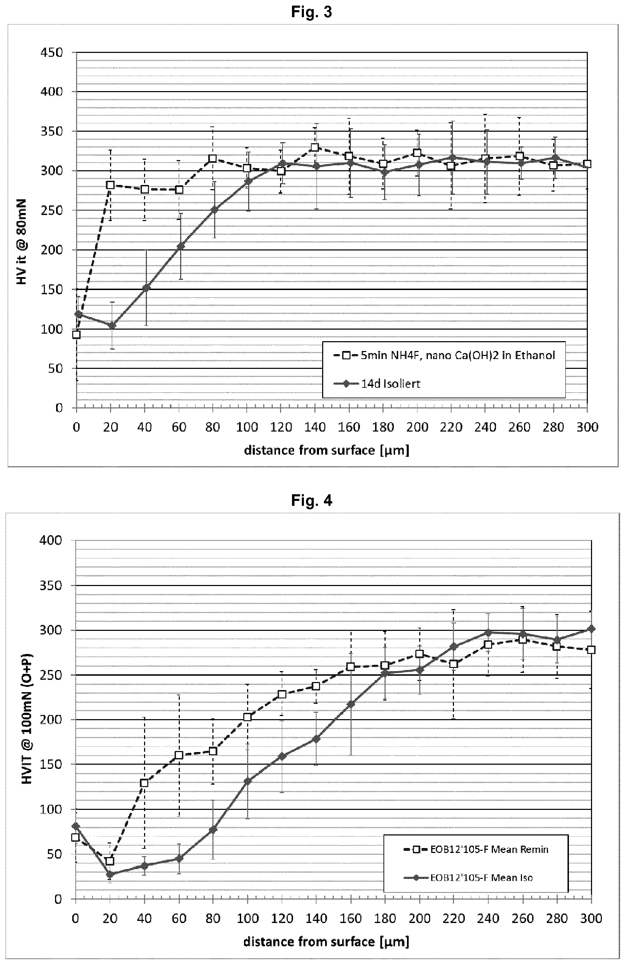

Fig. 3 zeigt das Härteprofil eines Zahnbereichs, der mit einer erfindungsgemäßen Zusammensetzung (NH₄F, danach ethanolisches Ca(OH)₂-Nanosol) behandelt wurde (gestrichelte Kurve), im Vergleich zu einer unbehandelten Schmelzprobe (durchgezogene Kurve). Die erfindungsgemäße Zusammensetzung bewirkt eine deutliche Zunahme der Härte in der demineralisierten Schmelzprobe. Die Härtezunahme ist auf die Remineralisation des Zahns zurückzuführen.
Fig. 4 zeigt das Härteprofil eines Zahnbereichs, der mit einer erfindungsgemäßen Zusammensetzung (NH₄F, danach ethanolisches CaCO₃-Nanosol) behandelt wurde (gestrichelte Kurve), im Vergleich zu einer unbehandelten Schmelzprobe (durchgezogene Kurve).

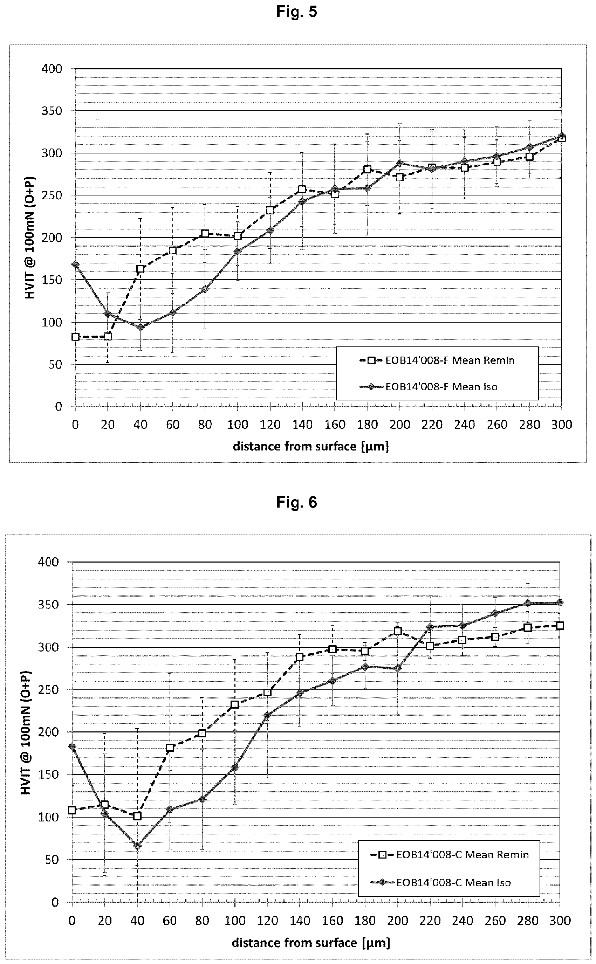

Fig. 5 zeigt das Härteprofil eines Zahnbereichs, der mit einer erfindungsgemäßen Zusammensetzung (NH₄F, danach ethanolisches CaF₂-Nanosol) behandelt wurde (gestrichelte Kurve), im Vergleich zu einer unbehandelten Schmelzprobe (durchgezogene Kurve). Die Proben wurden für 7 Tage in der demineralisierenden Lösung gelagert.
Fig. 6 zeigt das Härteprofil eines Zahnbereichs, der mit einer erfindungsgemäßen Zusammensetzung (NH₄F, danach ethanolisches CaF₂-Nanosol) behandelt wurde (gestrichelte Kurve), im Vergleich zu einer unbehandelten Schmelzprobe (durchgezogene Kurve). Die Proben wurden für 14 Tage in der demineralisierenden Lösung gelagert.

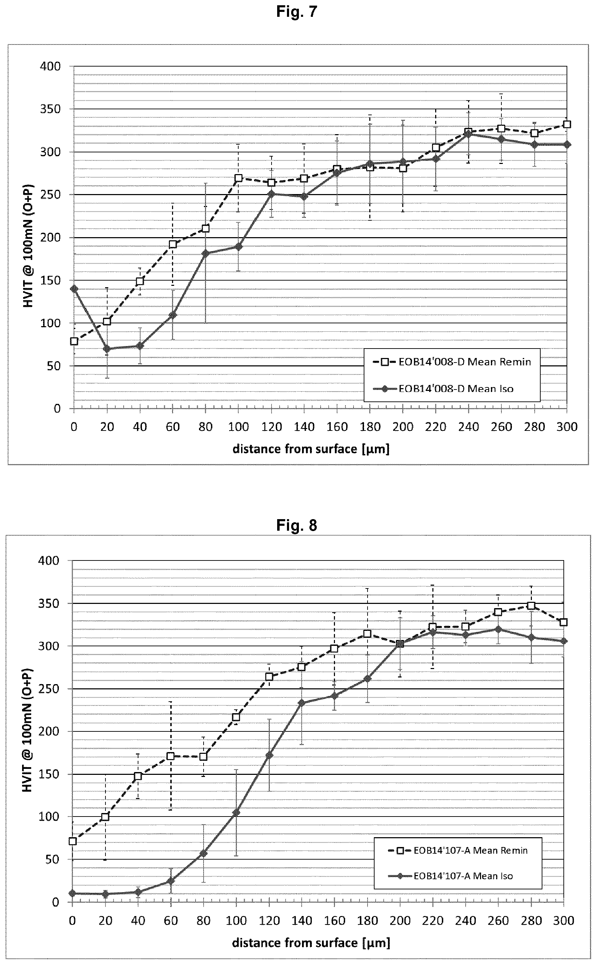

Fig. 7 zeigt das Härteprofil eines Zahnbereichs, der mit einer erfindungsgemäßen Zusammensetzung (NH₄F, danach ethanolisches CaF₂-Nanosol) behandelt wurde (gestrichelte Kurve), im Vergleich zu einer unbehandelten Schmelzprobe (durchgezogene Kurve). Die Proben wurden für 7 Tage in der demineralisierenden Lösung gelagert. Der CaF₂-Gehalt ist geringer als in Fig. 5.
Fig. 8 zeigt das Härteprofil eines Zahnbereichs, der mit einer erfindungsgemäßen Zusammensetzung (NH₄F, danach ethanolisches CaF₂-Nanosol) behandelt wurde (gestrichelte Kurve), im Vergleich zu einer unbehandelten Schmelzprobe (durchgezogene Kurve). Die Proben wurden für 14 Tage in der demineralisierenden Lösung gelagert. Der CaF₂-Gehalt ist geringer als in Fig. 6.

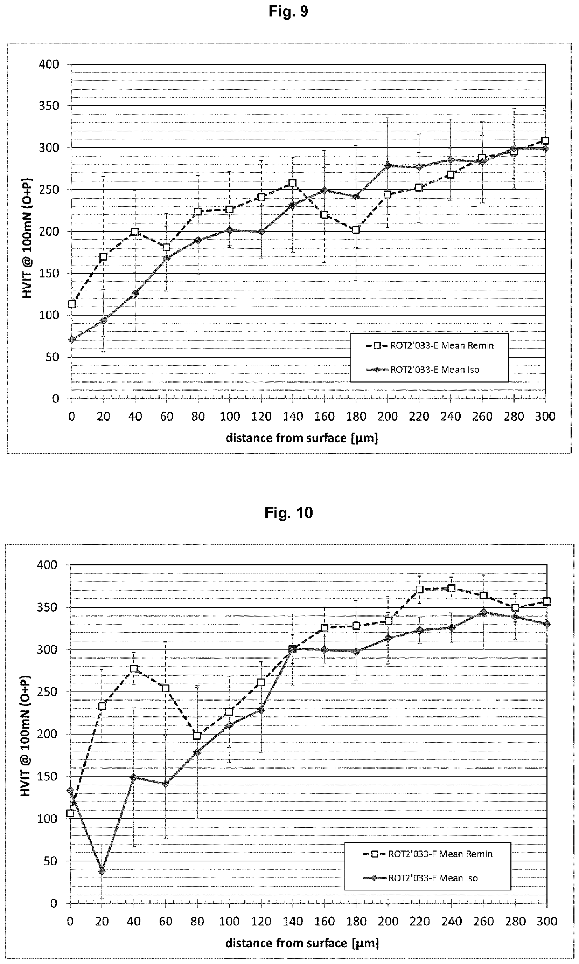

Fig. 9 zeigt das Härteprofil eines Zahnbereichs, der mit einer erfindungsgemäßen Zusammensetzung (NH₄HF₂, danach ethanolisches CaF₂-Nanosol) behandelt wurde (gestrichelte Kurve), im Vergleich zu einer unbehandelten Schmelzprobe (durchgezogene Kurve).
Fig. 10 zeigt das Härteprofil eines Zahnbereichs, der mit einer erfindungsgemäßen Zusammensetzung (Tetrabutylammoniumdihydrogentrifluorid, danach ethanolisches CaF₂-Nanosol) behandelt wurde (gestrichelte Kurve), im Vergleich zu einer unbehandelten Schmelzprobe (durchgezogene Kurve).

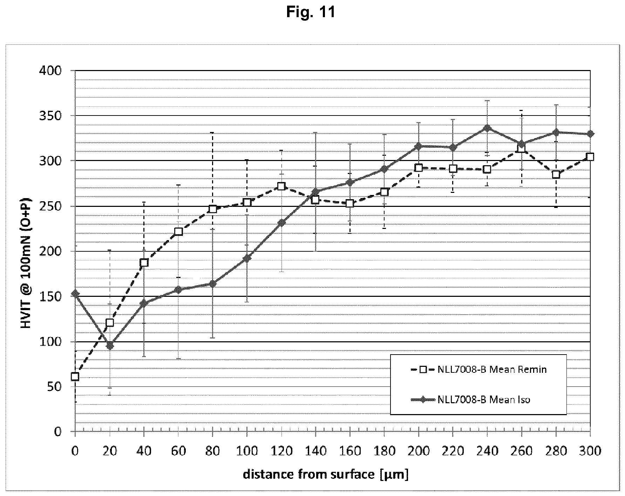

Fig. 11 zeigt das Härteprofil eines Zahnbereichs, der mit einer erfindungsgemäßen Zusammensetzung (NH₄F, danach wässriges CaF₂-Nanosol) behandelt wurde (gestrichelte Kurve), im Vergleich zu einer unbehandelten Schmelzprobe (durchgezogene Kurve). Die Proben wurden für 14 Tage in der demineralisierenden Lösung gelagert. Die Nanopartikel waren größer als in Figs. 6 und 8.

### Ausführungsbeispiele

### Beispiel 1

### Bestimmung des Remineralisationspotentials in kariogener Umgebung

Das Remineralisationspotential am Zahn wurde in einem Modell mit einer chemisch erzeugten Initialläsion in Rinderschmelz getestet. Dazu wurden Rinderzähne in Harz eingebettet, und mit SiC-Schleifpapier wurde unter Wasserkühlung der Schmelz freigelegt und poliert. Durch Lagerung für 14 bis 21 Tage bei 37°C in einer demineralisierenden Lösung wurde eine künstliche Läsion im Schmelz erzeugt. Die demineralisierende Lösung enthielt 50,0 mMol/l Essigsäure, 3,0 mMol/l KH₂PO₄, 3,0 mMol/l CaCl₂ · 2 H₂O, 1,0 ppm Methylendiphosphonsäure sowie als Konservierungsmittel noch 100 ppm Natriumazid. Der pH-Wert wurde mit KOH auf pH 5,0 eingestellt.

Die so erzeugten Läsionen wurden zur Hälfte mit Nagellack abgedeckt, die freie Fläche wurde mit dem erfindungsgemäßen Verfahren behandelt. Anschließend wurde der Prüfkörper bei 37°C für 7 - 14 Tage in der demineralisierenden Lösung bei pH 5,0 gelagert. Jede Behandlung wurde an je zwei Zähnen durchgeführt.

Nach der Lagerung wurden die Prüfkörper kurz mit Wasser abgespült, trockengetupft und der Nagellack der isolierten Hälfte mit Ethanol entfernt. Von beiden Hälften wurde mit einem Nanoindenter die Oberflächenhärte gemessen. Die Oberfläche der Prüfkörper wurde ebenfalls mit Harz eingebettet, um anschließend mit einer Diamantsäge eine Scheibe des Zahnquerschnitts aussägen zu können. Nach Polieren des Querschnitts wurden von der behandelten und isolierten Seite je drei Härteprofile vermessen (Eindrücke mit jeweils 20µm Abstand vertikal bis in eine Tiefe von 300µm, Berkovich-Indenter, 80 oder 100 mN Last, Beladung mit 400 oder 600 mN/Min., 2 s Haltezeit bei Fₘₐₓ). Mit den, vom Gerät automatisch berechneten, Vickershärtewerten wurden die Härteprofile erstellt.

In Fig. 1 wird das Härteprofil eines unbehandelten Zahns gezeigt. Es ist zu erkennen, dass die Härte des Zahn an der Oberfläche (distance = 0 µm) genau so hoch wie in tieferen Bereichen (distance = 300 µm) ist.

Fig. 2 zeigt das Härteprofil des Zahns nach Lagerung in der Demineralisationslösung. Hier ist die Härte an der Zahnoberfläche, die unmittelbar in Kontakt mit der Demineralisationslösung stand, deutlich verringert (von ca. 320 HV_{IT} auf ca. 40 HV_{IT}). Das Härteprofil zeigt, dass sich die Demineralisation bei den gewählten Bedingungen bis zu einer Tiefe von ca. 160 µm gemessen von der Zahnoberfläche auswirkt. Ab einer Tiefe von ca. 160 µm weist der Zahn die natürliche Härte auf.

### Beispiel 2

### Remineralisation mit Ammoniumfluoridlösuna und Calciumhvdroxid-Nanosol

Remineralisation einer Initialläsion durch Applikation einer 10%igen Ammoniumfluoridlösung (5,14 Gew.-% Fluorid) für 5 Minuten und anschließender Applikation eines Calciumhydroxid-Nanosols (50 g/l in Ethanol, absoluter Korngrößenbereich 50 - 250 nm; erhalten von IBZ-Salzchemie GmbH & Co. KG, 09633 Halsbrücke, Deutschland). Nach Eintrocknen des Sols wurden die Zähne für 14 Tage in der demineralisierenden Lösung gelagert.

Die Härteprofile dieser Probe sind in Fig. 3 wiedergegeben. Die durchgehende Linie zeigt das Profil der mit Nagellack isolierten Hälfte der Schmelzproben. Durch die Demineralisation ist die Härte der Schmelzprobe im Oberflächenbereich deutliche verringert (von ca. 300 HV_{IT} auf ca. 100 HV_{IT}).

Die gestrichelte Kurve zeigt das Härteprofil der Schmelzprobe, die mit der erfindungsgemäßen Zusammensetzung behandelt und anschließend erneut der Demineralisationslösung ausgesetzt wurde. Die Behandlung hat eine sehr deutliche Erhöhung der Härte im Oberflächenbereich und damit eine Remineralisation des Zahns bewirkt.

### Beispiel 3

### Remineralisation mit Ammoniumfluoridlösung und Calciumcarbonat-Nanosol (6 %)

Remineralisation einer Initialläsion durch Applikation einer 10%igen Ammoniumfluoridlösung (5,14 Gew.-% Fluorid) für 5 Minuten und anschließender Applikation eines Calciumcarbonat-Nanosols (6 Gew.-% in Ethanol, mittlerer Partikeldurchmesser gemessen mit dynamischer Lichtstreuung (DLS) von ca. 70 nm; erhalten von Mathym SAS, 69410 Champagne-au-Mont-d'Or, Frankreich). Nach Eintrocknen des Sols wurden die Zähne für 9 Tage in der demineralisierenden Lösung gelagert.

Die Härteprofile in Fig. 4 zeigen, dass die Behandlung mit der erfindungsgemäßen Lösung eine deutliche Härtezunahme bewirkt.

### Beispiel 4

### Remineralisation mit Ammoniumfluoridlösung/Calciumfluorid-Nanosol (10,6 %, 7 Tage)

Remineralisation einer Initialläsion durch Applikation einer 10%igen Ammoniumfluoridlösung (5,14 Gew.-% Fluorid) für 5 Minuten und anschließender Applikation eines Calciumfluorid-Nanosols (10.6 Gew.-% in Ethanol, mittlerer Partikeldurchmesser gemessen mit dem Transmissionselektronenmikroskop (TEM) von ca. 20 nm, oder mit DLS von ca. 30 nm; erhalten von Mathym SAS, 69410 Champagne-au-Mont-d'Or, Frankreich). Nach Eintrocknen des Sols wurden die Zähne für 7 Tage in der demineralisierenden Lösung gelagert.

Die Härteprofile der Proben werden in Fig. 5 gezeigt. Es ist eine deutliche Härtezunahme bei der erfindungsgemäß behandelten Probe zu erkennen.

### Beispiel 5

### Remineralisation mit Ammoniumfluoridlösung/Calciumfluorid-Nanosol (10,6 %, 14 Tage)

Remineralisation einer Initialläsion durch Applikation einer 10%igen Ammoniumfluoridlösung (5,14 Gew.-% Fluorid) für 5 Minuten und anschließender Applikation eines Calciumfluorid-Nanosols (10.6 Gew.-% in Ethanol, mittlerer Partikeldurchmesser gemessen mit TEM von ca. 20 nm, mit DLS von ca. 30 nm; erhalten von Mathym SAS, 69410 Champagne-au-Mont-d'Or, Frankreich). Nach Eintrocknen des Sols wurden die Zähne für 14 Tage in der demineralisierenden Lösung gelagert.

Die Härteprofile in Fig. 6 belegen eine wirksame Remineralisation des Zahns.

### Beispiel 6

### Remineralisation mit Ammoniumfluoridlösung und Calciumfluorid-Nanosol (6,5 %)

Remineralisation einer Initialläsion durch Applikation einer 10%igen Ammoniumfluoridlösung (5,14 Gew.-% Fluorid) für 5 Minuten und anschließender Applikation eines Calciumfluorid-Nanosols (6,5 Gew.-% in Ethanol, mittlerer Partikeldurchmesser gemessen mit TEM von ca. 10 nm, mit DLS von ca. 30 nm; erhalten von Mathym SAS, 69410 Champagne-au-Mont-d'Or, Frankreich). Nach Eintrocknen des Sols wurden die Zähne für 7 Tage in der demineralisierenden Lösung gelagert.

Die Härteprofile der Proben werden in Fig. 7 gezeigt. Sie belegen eine wirksame Remineralisation des Zahns.

### Beispiel 7

### Remineralisation mit Ammoniumfluoridlösung und Calciumfluorid-Nanosol (7,1 %)

Remineralisation einer Initialläsion durch Applikation einer 10%igen Ammoniumfluoridlösung (5,14 Gew.-% Fluorid) für 5 Minuten und anschließender Applikation eines Calciumfluorid-Nanosols (7,1 Gew.-% in Ethanol, mittlerer Partikeldurchmesser gemessen mit TEM von ca. 10 nm, ca. 30 nm mit DLS; erhalten von Mathym SAS, 69410 Champagne-au-Mont-d'Or, Frankreich). Nach Eintrocknen des Sols wurden die Zähne für 14 Tage in der demineralisierenden Lösung gelagert.

Die Härteprofile in Fig. 8 zeigen eine sehr deutliche Zunahme der Härte nach der Remineralisation des Zahns.

### Beispiel 8

### Remineralisation mit Ammoniumbifluoridlösung/Calciumfluorid-Nanosol

Remineralisation einer Initialläsion durch Applikation einer 3,85%igen Lösung Ammoniumbifluorid NH₄HF₂ (2,56 Gew.-% Fluorid) für 1 Minute und anschließender Applikation eines Calciumfluorid-Nanosols (7,1 Gew.-% in Ethanol, mittlerer Partikeldurchmesser gemessen mit TEM von ca. 10 nm, ca. 30 nm mit DLS; erhalten von Mathym SAS, 69410 Champagne-au-Mont-d'Or, Frankreich). Nach Eintrocknen des Sols wurden die Zähne für 14 Tage in der demineralisierenden Lösung gelagert.

Die Härteprofile in Fig. 9 zeigen eine deutliche, wenn auch etwas weniger stark ausgeprägte Härtezunahme nach der Remineralisation als die übrigen Beispiele.

### Beispiel 9

### Remineralisation mit Tetrabutylammoniumdihydrogentrifluorid/Calciumfluorid-Nanosol

Remineralisation einer Initialläsion durch Applikation einer 13,58%igen Lösung von Tetrabutylammoniumdihydrogentrifluorid in Wasser (2,56 Gew.-% Fluorid) für 1 Minute und anschließender Applikation eines Calciumfluorid-Nanosols (7,1 Gew.-% in Ethanol, mittlerer Partikeldurchmesser gemessen mit TEM von ca. 10 nm, mit DLS von ca. 30 nm; erhalten von Mathym SAS, 69410 Champagne-au-Mont-d'Or, Frankreich). Nach Eintrocknen des Sols wurden die Zähne für 14 Tage in der demineralisierenden Lösung gelagert.

Die Härteprofile in Fig. 10 zeigen eine sehr deutliche Zunahme der Härte nach der Remineralisation des Zahns.

### Beispiel 10

### Remineralisation mit Ammoniumfluoridlösung/Calciumfluorid-Nanosol (8,2 %)

Remineralisationsversuch einer Initialläsion durch Applikation einer 10%igen Ammoniumfluoridlösung (5,14 Gew.-% Fluorid) für 5 Minuten und anschließender Applikation eines Calciumfluorid-Nanosols (8,2 Gew.-% in Wasser, mittlerer Partikeldurchmesser gemessen mit DLS von ca. 60 nm; erhalten von Transparent Materials LLC, Rochester, NY 14615, USA). Nach Eintrocknen des Sols wurden die Zähne für 14 Tage in der demineralisierenden Lösung gelagert.

Die Härteprofile in Fig. 11 zeigen eine Zunahme der Härte nach der Remineralisation des Zahns.

## Patentansprüche

1. Zusammensetzung zur Verwendung bei der Remineralisation von Zähnen, die eine fluoridhaltige Komponente und eine calciumhaltige Komponente umfasst, **dadurch gekennzeichnet, dass** die calciumhaltige Komponente ein nanopartikuläres Calciumsalz enthält und dass die Zusammensetzung zur aufeinanderfolgenden Applikation der fluoridhaltigen Komponente und der calciumhaltigen Komponente auf die Zahnoberfläche hergerichtet ist, wobei die fluoridhaltige Komponente vor der calciumhaltigen Komponente auf den Zahn aufgebracht wird.

2. Zusammensetzung zur Verwendung gemäß Anspruch 1, wobei die calciumhaltige Komponente nach dem Aufbringen auf die Zahnoberfläche eine feste Schicht bildet.

3. Zusammensetzung zur Verwendung gemäß Anspruch 1 oder 2 bei der Behandlung initialer Kariesläsionen, zum Kariesschutz, zur Verhinderung und/oder Behandlung der Zahnerosion.

4. Zusammensetzung zur Verwendung gemäß einem der vorhergehenden Ansprüche, wobei die fluoridhaltige Komponente Natriumfluorid, Kaliumfluorid, Ammoniumfluorid, Ammoniumbifluorid, Natriummonofluorophosphat, Kaliummonofluorophosphat, Salze von Tetra- oder Hexafluoroanionen wie z.B. Ammoniumhexafluorosilikat, Magnesiumhexafluorosilikat, Kaliumhexafluorophosphat, Ammoniumhexafluorotitanat, Ammoniumhexafluoroaluminat, Zirkonfluorid, Tetra-n-butylammonium dihydrogentrifluorid (TBAF-3), Rubidiumfluorid, Cäsiumfluorid, Kaliumbifluorid (KHF₂), Silber-I-fluorid (AgF), Zinn-II-fluorid (SnF₂), Olaflur oder Dectaflur oder eine Mischung davon enthält, vorzugsweise Ammoniumfluorid, Ammoniumbifluorid, Kaliumfluorid und/oder Tetra-n-butylammoniumdihydrogentrifluorid.

5. Zusammensetzung zur Verwendung gemäß einem der vorangehenden Ansprüche, wobei die fluoridhaltige Komponente eine Lösung eines Fluorids in einem Lösungsmittel enthält, vorzugsweise in Wasser, Ethanol, Isopropanol, Aceton, Methanol und Propylenglycol oder einer Mischungen davon.

6. Zusammensetzung zur Verwendung gemäß einem der vorangehenden Ansprüche, wobei die fluoridhaltige Komponente mehr als 1500 ppm, bevorzugt mehr als 5000 ppm Fluorid (bezogen auf das Fluoridanion) in gelöster Form enthält.

7. Zusammensetzung zur Verwendung gemäß Anspruch 6, wobei die fluoridhaltige Komponente weniger als 20 Gew.-% und vorzugsweise weniger als 10 Gew.-% Fluorid enthält.

8. Zusammensetzung zur Verwendung gemäß einem der vorangehenden Ansprüche, wobei die fluoridhaltige Komponente zusätzlich eine Säure enthält, vorzugsweise Phosphorsäure, Salzsäure, Salpetersäure, Schwefelsäure, Milchsäure, Essigsäure, Ameisensäure, Zitronensäure oder Ethylendiamintetraessigsäure.

9. Zusammensetzung zur Verwendung gemäß einem der vorangehenden Ansprüche, wobei die calciumhaltige Komponente Calciumfluorid, Calciumcarbonat, Calciumsulfat, Calciumsilikat, Calciumoxid oder Calciumhydroxid enthält.

10. Zusammensetzung zur Verwendung gemäß Anspruch 9, wobei die calciumhaltige Komponente Calciumfluorid, Calciumcarbonat, Calciumsulfat und/oder Calciumsilikat, vorzugsweise Calciumcarbonat und insbesondere Calciumfluorid enthält.

11. Zusammensetzung zur Verwendung gemäß einem der vorangehenden Ansprüche, wobei das nanopartikuläre Calciumsalz einen mittleren Partikeldurchmesser von < 100 nm, vorzugsweise von < 30 nm aufweist, gemessen durch dynamische Lichtstreuung.

12. Zusammensetzung zur Verwendung gemäß einem der vorangehenden Ansprüche, wobei das nanopartikuläre Calciumsalz in einem Lösungsmittel suspendiert ist, vorzugsweise in einem Alkohol, Ester, Ether, Keton, Alkan, Alken, Wasser oder einer Mischung davon, vorzugsweise in Ethanol, Methanol, n-Propanol, i-Propanol, n-Butanol, sek. Butanol, Isoamylalkohol, Aceton, Wasser, Acetonitril, Ethylacetat, Methoxypropanol, Dibutylether, Dioxan, Metylethylketon, Heptan, Hexan oder Dimethylformamid.

13. Zusammensetzung zur Verwendung gemäß Anspruch 12, wobei das nanopartikuläre Calciumsalz in Ethanol, Aceton, i-Propanol, Wasser oder einer Mischung davon suspendiert ist.

14. Zusammensetzung zur Verwendung gemäß Anspruch 12 oder 13, wobei die Zusammensetzung 0,1 Gew.-% bis 40 Gew.-%, besonders bevorzugt 1 Gew.-% bis 30 Gew.-% und ganz besonders bevorzugt 5 Gew.-% bis 25 Gew.-% nanopartikuläres Calciumsalz enthält.

15. Zusammensetzung zur Verwendung gemäß einem der vorherigen Ansprüche, wobei die Zusammensetzung zusätzlich eine Säurelösung zum Anätzen des oder der Zähne enthält und die zum Aufbringen der Säurelösung auf die Zahnoberfläche vor der fluoridhaltigen Komponente hergerichtet ist.

## Claims

1. Composition for use in the remineralization of teeth, which comprises a fluoride-containing component and a calcium-containing component, **characterized in that** the calcium-containing component comprises a nanoparticulate calcium salt and **in that** the composition is designed for the successive application of the fluoride-containing component and the calcium-containing component to the tooth surface, wherein the fluoride-containing component is applied to the tooth before the calcium-containing component.

2. Composition for use according to claim 1, wherein the calcium-containing component forms a solid layer after being applied to the tooth surface.

3. Composition for use according to claim 1 or 2 in the treatment of initial caries lesions, for caries protection, for the prevention and/or treatment of dental erosion.

4. Composition for use according to one of the preceding claims, wherein the fluoride-containing component comprises sodium fluoride, potassium fluoride, ammonium fluoride, ammonium bifluoride, sodium monofluorophosphate, potassium monofluorophosphate, salts of tetra- or hexafluoro anions such as ammonium hexafluorosilicate, magnesium hexafluorosilicate, potassium hexafluorophosphate, ammonium hexafluorotitanate, ammonium hexafluoroaluminate, zirconium fluoride, tetra-n-butylammonium dihydrogen trifluoride (TBAF-3), rubidium fluoride, cesium fluoride, potassium bifluoride (KHF₂), silver(I) fluoride (AgF), tin(II) fluoride (SnF₂), olaflur or dectaflur or a mixture thereof, preferably ammonium fluoride, ammonium bifluoride, potassium fluoride and/or tetra-n-butylammonium dihydrogen trifluoride.

5. Composition for use according to one of the preceding claims, wherein the fluoride-containing component comprises a solution of a fluoride in a solvent, preferably in water, ethanol, isopropanol, acetone, methanol and propylene glycol or a mixture thereof.

6. Composition for use according to one of the preceding claims, wherein the fluoride-containing component comprises more than 1500 ppm, preferably more than 5000 ppm, fluoride (relative to the fluoride anion) in dissolved form.

7. Composition for use according to claim 6, wherein the fluoride-containing component comprises less than 20 wt.-% and preferably less than 10 wt.-% fluoride.

8. Composition for use according to one of the preceding claims, wherein the fluoride-containing component additionally comprises an acid, preferably phosphoric acid, hydrochloric acid, nitric acid, sulfuric acid, lactic acid, acetic acid, formic acid, citric acid or ethylenediaminetetraacetic acid.

9. Composition for use according to one of the preceding claims, wherein the calcium-containing component comprises calcium fluoride, calcium carbonate, calcium sulphate, calcium silicate, calcium oxide or calcium hydroxide.

10. Composition for use according to claim 9, wherein the calcium-containing component comprises calcium fluoride, calcium carbonate, calcium sulphate and/or calcium silicate, preferably calcium carbonate and in particular calcium fluoride.

11. Composition for use according to one of the preceding claims, wherein the nanoparticulate calcium salt has an average particle diameter of < 100 nm, preferably of < 30 nm, measured by dynamic light scattering.

12. Composition for use according to one of the preceding claims, wherein the nanoparticulate calcium salt is suspended in a solvent, preferably in an alcohol, ester, ether, ketone, alkane, alkene, water or a mixture thereof, preferably in ethanol, methanol, n-propanol, i-propanol, n-butanol, sec-butanol, isoamyl alcohol, acetone, water, acetonitrile, ethyl acetate, methoxy propanol, dibutyl ether, dioxane, methyl ethyl ketone, heptane, hexane or dimethylformamide.

13. Composition for use according claim 12, wherein the nanoparticulate calcium salt is suspended in ethanol, acetone, i-propanol, water or a mixture thereof.

14. Composition for use according to claim 12 or 13, wherein the composition comprises 0.1 wt.-% to 40 wt.-%, particularly preferably 1 wt.-% to 30 wt.-% and quite particularly preferably 5 wt.-% to 25 wt.-%, nanoparticulate calcium salt.

15. Composition for use according to one of the preceding claims, wherein the composition additionally comprises an acid solution for etching the tooth or teeth and which is designed for the application of the acid solution to the tooth surface before the fluoride-containing component.

## Revendications

1. Composition pour utilisation dans la reminéralisation des dents, comprenant un composant contenant un fluorure et un composant contenant du calcium, **caractérisée en ce que** le composant contenant du calcium contient un sel de calcium nanoparticulaire et **en ce que** la composition est préparée pour que le composant contenant un fluorure et le composant contenant du calcium soient appliqués successivement sur la surface d'une dent, étant entendu que le composant contenant un fluorure est appliqué sur la dent avant le composant contenant du calcium.

2. Composition pour utilisation conforme à la revendication 1, de laquelle le composant contenant du calcium forme une couche solide après application sur la surface d'une dent.

3. Composition pour utilisation, conforme à la revendication 1 ou 2, dans le traitement de lésions carieuses initiales, pour protection contre les caries, ou pour empêcher et/ou traiter l'érosion dentaire.

4. Composition pour utilisation conforme à l'une des revendications précédentes, dans laquelle le composant contenant un fluorure contient du fluorure de sodium, du fluorure de potassium, du fluorure d'ammonium, du bifluorure d'ammonium, du monofluorophosphate de sodium, du monofluorophosphate de potassium, des sels d'anions tétra-fluorés ou hexafluorés comme par exemple de l'hexafluorosilicate d'ammonium, de l'hexafluorosilicate de magnésium, de l'hexafluorophosphate de potassium, de l'hexafluorotitanate d'ammonium ou de l'hexafluoroaluminate d'ammonium, du fluorure de zirconium, du dihydrogénotrifluorure de tétra-n-butyl-ammonium (TBAF-3), du fluorure de rubidium, du fluorure de césium, du bifluorure de potassium (KHF₂), du fluorure d'argent-1 (AgF), du fluorure d'étain-II (SnF₂), de l'Olaflur ou du Dectaflur, ou un mélange de ceux-ci, et de préférence du fluorure d'ammonium, du bifluorure d'ammonium, du fluorure de potassium et/ou du dihydrogénotrifluorure de tétra-n-butyl-ammonium.

5. Composition pour utilisation conforme à l'une des revendications précédentes, dans laquelle le composant contenant un fluorure contient une solution d'un fluorure dans un solvant, de préférence dans de l'eau, de l'éthanol, de l'isopropanol, de l'acétone, du méthanol ou du propylèneglycol, ou dans un mélange de ces solvants.

6. Composition pour utilisation conforme à l'une des revendications précédentes, dans laquelle le composant contenant un fluorure contient plus de 1500 ppm, et de préférence plus de 5000 ppm, de fluorure (exprimé en anion fluorure) à l'état dissous.

7. Composition pour utilisation conforme à la revendication 6, dans laquelle le composant contenant un fluorure contient moins de 20 % en poids, et de préférence moins de 10 % en poids, de fluorure.

8. Composition pour utilisation conforme à l'une des revendications précédentes, dans laquelle le composant contenant un fluorure contient en outre un acide, et de préférence, de l'acide phosphorique, de l'acide chlorhydrique, de l'acide nitrique, de l'acide sulfurique, de l'acide lactique, de l'acide acétique, de l'acide formique, de l'acide citrique ou de l'acide éthylènediamine-tétraacétique.

9. Composition pour utilisation conforme à l'une des revendications précédentes, dans laquelle le composant contenant du calcium contient du fluorure de calcium, du carbonate de calcium, du sulfate de calcium, du silicate de calcium, de l'oxyde de calcium, ou de l'hydroxyde de calcium.

10. Composition pour utilisation conforme à la revendication 9, dans laquelle le composant contenant du calcium contient du fluorure de calcium, du carbonate de calcium, du sulfate de calcium et/ou du silicate de calcium, de préférence du carbonate de calcium, et en particulier du fluorure de calcium.

11. Composition pour utilisation conforme à l'une des revendications précédentes, dans laquelle le sel de calcium nanoparticulaire présente un diamètre moyen de particule, mesuré par diffusion dynamique de la lumière, de moins de 100 nm, et de préférence, de moins de 30 nm.

12. Composition pour utilisation conforme à l'une des revendications précédentes, dans laquelle le sel de calcium nanoparticulaire est en suspension dans un solvant, de préférence dans un alcool, un ester, un éther, une cétone, un alcane, un alcène ou de l'eau, ou dans un mélange de tels composés, et de préférence dans de l'éthanol, du méthanol, du n-propanol, de l'isopropanol, du n-butanol, du sec-butanol, de l'alcool isoamylique, de l'acétone, de l'eau, de l'acétonitrile, de l'acétate d'éthyle, du méthoxy-propanol, du dibutyl-éther, du dioxane, de la méthyl-éthyl-cétone, de l'heptane, de l'hexane, ou du diméthyl-formamide.

13. Composition pour utilisation conforme à la revendication 12, dans laquelle le sel de calcium nanoparticulaire est en suspension dans de l'éthanol, de l'acétone, de l'isopropanol ou de l'eau, ou dans un mélange de ceux-ci.

14. Composition pour utilisation conforme à la revendication 12 ou 13, laquelle composition contient de 0,1 % en poids à 40 % en poids, en particulier de 1 % en poids à 30 % en poids, et tout particulièrement de 5 % en poids à 25 % en poids, de sel de calcium nanoparticulaire.

15. Composition pour utilisation conforme à l'une des revendications précédentes, laquelle composition contient en outre une solution d'acide pour le mordançage de la ou des dent(s), et est préparée pour que la solution d'acide soit appliquée sur la surface de la ou des dent(s) avant le composant contenant un fluorure.
